Europäisches Patentamt

European Patent Office

Office européen des brevets

(11) Publication number: **0 071 623**
**B1**

## EUROPEAN PATENT SPECIFICATION

(45) Date of publication of patent specification: **15.10.86**

(51) Int. Cl.⁴: **A 23 K 1/14**

(21) Application number: **82900502.4**

(22) Date of filing: **09.02.82**

(86) International application number:
**PCT/DK82/00013**

(87) International publication number:
**WO 82/02650 19.08.82 Gazette 82/20**

(54) Method of promoting growth in monogastric animals, poultry and ruminants.

(30) Priority: **10.02.81 DK 555/81**

(43) Date of publication of application:
**16.02.83 Bulletin 83/07**

(45) Publication of the grant of the patent:
**15.10.86 Bulletin 86/42**

(84) Designated Contracting States:
**BE DE FR GB NL**

(56) References cited:
**FR-A-1 461 347**
**US-A-2 060 336**
**US-A-4 120 952**

**Remington's Pharmaceutical Sciences, 15th ed. 1975, pages 744, 746**

**The extra pharmacopoeia, 26th ed, (1972), page 1084**

**Natural Gums, Chap. XIV, pages 345-354**

**The file contains technical information submitted after the application was filed and not included in this specification**

(73) Proprietor: **Von MAGIUS, Niels Wilhelm**
**Langövägen 7**
**S-253 72 Helsingborg (SE)**

(72) Inventor: **Von MAGIUS, Niels Wilhelm**
**Langövägen 7**
**S-253 72 Helsingborg (SE)**

Courier Press, Leamington Spa, England.

EP 0 071 623 B1

## Description

The present invention concerns a method of promoting growth in monogastric animals, poultry and ruminants.

It has been found that Plantago species in the form of seed husk, whole or ground seeds or extractions thereof, used as an additive to animal feeds, gives an extremely favourable result in obtaining a growth-promoting effect, especially in monogastric animals, such as pigs, horses, minks, dogs, cats and other pets, as well as poultry, but also in ruminants, such as cattle and sheep both at the stage when they only have one active stomach (e.g. calves and lambs) and when they are full-grown (e.g. cows and sheep).

Plantago seeds are widely used as a human laxative in various dosage forms and have for centuries been used as a remedy against a large number of human diseases, such as inflammation of the eye, dry coughs, burns and certain gastric and intestinal disorders (see e.g. Martindale, The Extra Pharmacopoeia 26th ed., p. 1084 and J. N. BeMiller: Natural Gums, chapter XVI, pp. 345—354).

The effect of Plantago seeds is due to the content of gum, which can be readily extracted with hot water. It is, however, not necessary to extract the gum since the seeds can in ground form be used directly because of their great mucilage-forming capacity.

A preferred Plantago product is Ispaghula (BPC) because this has the highest content of polysaccharide. Ispaghula consists of the dried, ripe seeds of Plantago ovata Forskal. In the USP the designation "Psyllium seeds" covers both Plantago ovata, P. indica and P psyllium seeds. In Denmark the name "loppefrφ" (flea seeds) only covers P. psyllium and P. indica. The main constituents of Ispaghula are polysaccharides (about 30%), composed of D-xylose, L'arabinose, L'rhamnose and D-galacturonic acid. Ispaghula Husk is the name of epidermis and collapsed adjacent layers removed from Ispaghula.

The polysaccharide content is higher than in Ispaghula. Therefore and because the outer seed embryo contains a pigment that causes a brown coloration of the kidneys, the use of Ispaghula Husk is preferred to the whole seeds.

Among the physical properties of Ispaghula Husk, the most remarkable one is its ability to absorb water under formation of a highly viscous mucilage. A dispersion of 1 percent Ispaghula Husk in water gives a viscosity of 4000 mPas. The dispersions are thixotropic. The viscosity is only slightly affected by temperature variations between 20 and 50°C, pH variations between 2 and 10 and electrolyte solutions up to 0.2 M (sodium and potassium chloride).

Ispaghula Husk, when ingested by animals with the proper amount of water, swells and becomes an integral part of the fecal mass. Ingestion of 8 g of Ispaghula Husk is able to increase the fecal mass by more than 200 ml owing to the water bound.

Ispaghula Husk has no nutritional value and is not broken down during the passage through the intestinal tract of the animal. The substance binds toxines and allergens.

Ispaghula Husk has a plurality of advantages which make the substance useful:

— it is a safe and harmless natural product which is widely used in humans,

— it does not alter the normal bacterial flora, neither in the intestines nor in the excrements,

— it does not change iron and calcium resorption,

— it is heat resistant and is not broken down in boiling water, thus it can be easily formulated in pellets; in this context it acts as a binder and prevents dust formation,

— its properties do not change by the addition of NaCl or KCl, even in the presence of citric acid (this is essential in the treatment of severe diarrhoea, where such treatment is given with potassium ions),

— the treatment is simple and inexpensive and requires no veterinary assistance, and

— because of its great water-binding capacity, it exerts a hydrostatic effect on the water balance between the intestine and the surrounding cells, counteracting the liberation of water from the cells in consequence of stress.

The recommended dose is low, normally of the order of 0.3% by weight mixed with the animal feed or of the order of 0.5 g per kg of body weight. The dose is, however, not critical; large doses are well tolerated.

The preferred product for the purpose of the invention has, as mentioned, its origin in the Plantago ovata species, but even products originating in other Plantago species, such as P. psyllium, P. indica, P. lanceolata and P. major, can be used with similarly good results.

The following example illustrates more closely the effect obtained by using the preferred product, Ispaghula Husk.

## Example

A test with 20 day old weanlings of excellent health was carried out. The piglets were placed in steel pens and separated in two groups: Control (C) and Test (T) with 40 piglets in each. The average weight in each group was five kg. Feed and water were available on a free choice basis.

The control group was given a standard feed for weanlings. The test group was given Ispaghula Husk mixed with a standard feed for weanlings in the ratio of 3 kg Ispaghula Husk to 1000 kg standard feed.

The total incidence of scours was designated S. The watery scours were designated WS. The average feed consumption F and the average growth G for each group during the first three weeks and the following two weeks of the test period were recorded.

Test results

The incidence of scours (weekly average per 100 animals) appears from Table I.

TABLE I

| Week | T—S | T—Ws | C—S | C—WS |
|------|-----|------|-----|------|
| 1 | 65 | 19 | 89 | 19 |
| 2 and 3 | 155 | 72 | 254 | 149 |
| 4 and 5 | 94 | 30 | 107 | 29 |

During the first week there were no significant differences between the two groups, the incidence of scours being low overall.

During the second week S/WS increased rapidly in the C group to a maximum of 260/155.

During the third week the two groups remained at the same level as in the previous week.

During the fourth week S/WS declined rapidly to 105/28 in the C group, whereas it took the T group two days more to arrive at 90/25.

The levels remained unchanged in the fifth week.

TABLE II
Average feed consumption and growth in the test period:

| Week | T—F | T—G | C—F | C—G |
|------|-----|-----|-----|-----|
| 0—3 | 7.27 | 5.75 | 7.14 | 5.53 |
| 4—5 | 10.75 | 7.48 | 10.57 | 7.03 |

TABLE III
Feed consumption to obtain a growth of 1 kg:

| Week | $F_G^F$ | $C_G^F$ |
|------|---------|---------|
| 0—3 | 1.264 | 1.290 |
| 4—5 | 1.432 | 1.504 |

TABLE IV
Difference in the feed consumption per 1 kg growth between the two groups:

| Week | T/C |
|------|-----|
| 0—3 | 0.98 |
| 4—5 | 0.95 |

The effect of Ispaghula Husk admixture to the feed can—in cases where no drastic diarrhoea occurs—be measured best from the amount of feed necessary to obtain a growth of 1 kg. As appears from the above figures, the test animals used 2 percent less feed during the first three weeks and 5 percent less feed during the weeks 4 and 5 than the control animals.

## Claims

1. A method of promoting growth in monogastric animals, poultry and ruminants, characterized by administering to the animal an effective amount of a feed additive based on a Plantago species.

2. A method according to claim 1, characterized by the fact that the Plantago species employed is P. ovata, P. psyllium, P. indica, P. lanceolata or P. major.

3. A method according to claim 1 or 2, characterized by the fact that the product employed is Ispaghula, preferably Ispaghula Husk.

4. A method according to claim 1, 2 or 3, characterized by the fact that the animal is fed a feed containing about 0.3 percent by weight of Ispaghula Husk.

5. A method according to claim 1, 2 or 3, characterized by the fact that the animal is fed a feed containing Ispaghula Husk equivalent to 0.5 g per kg of body weight.

## Patentansprüche

1. Verfahren zur Wachstumsförderung in monogastrischen Tieren, Geflügel und Wiederkäuern, dadurch gekennzeichnet dass dem Tier eine wirkungsvolle Menge eines auf einer Plantago-Art basierten Futterzusatzes gegeben wird.

2. Verfahren nach Anspruch 1, dadurch gekennzeichnet dass die verwendete Plantago-Art P. ovata, P. psyllium, P. indica, P. lanceolata oder P. major ist.

3. Verfahren nach Anspruch 1 oder 2, dadurch gekennzeichnet dass das verwendete Produkt Ispaghula, vorzugsweise Ispaghula Husk, ist.

4. Verfahren nach einem der Ansprüche 1, 2 oder 3, dadurch gekennzeichnet dass das Tier mit einem Futter, das etwa 0,3 Gewichtsprozent Ispaghula Husk enthält, gefüttert wird.

5. Verfahren nach einem der Ansprüche 1, 2 oder 3, dadurch gekennzeichnet dass das Tier mit einem Futter, das Ispaghula Husk gleichwertig mit 0,5 g per kg des Körpergewichts enthält, gefüttert wird.

## Revendications

1. Une méthode pour favoriser la croissance des animaux monogastriques, de la volaille et des ruminants, caractérisée par l'administration à l'animal d'une quantité efficace d'additif alimentaire basé sur un espèce de Plantago.

2. Une méthode selon la revendication, caractérisée en ce que l'espèce de Plantago employée est P. ovata, P. psyllium, P. indica, P. lanceolata ou P. major.

3. Une méthode selon l'une des revendications 1 ou 2, caractérisée en ce que le produit employé est Ispaghula, de préférence Ispaghula Husk.

4. Une méthode selon l'une des revendications 1, 2 ou 3, caractérisée en ce que l'animal est nourri d'une nourriture qui contient environ 0,3 pourcent en poids d'Ispaghula Husk.

5. Une méthode selon l'une des revendications 1, 2 ou 3, caractérisée en ce que l'animal est nourri d'une nourriture qui contient Ispaghula Husk équivalent à 0,5 g par kg du poids du corps.